# EUROPÄISCHE PATENTSCHRIFT

(11) **EP 1 194 827 B1**
(45) Veröffentlichungstag und Bekanntmachung des Hinweises auf die Patenterteilung: **12.11.2003**
(21) Anmeldenummer: 00943821.9
(22) Anmeldetag: 16.06.2000
(51) Int. Cl.: G06F 19/00

(54) **VERFAHREN UND SYSTEM ZUM DARSTELLEN CHEMISCHER STRUKTURFORMELN**
METHOD AND SYSTEM FOR REPRESENTING CHEMICAL STRUCTURAL FORMULAE
PROCEDE ET SYSTEME PERMETTANT DE REPRESENTER DES FORMULES DE STRUCTURES CHIMIQUES

(30) Priorität: 22.06.1999 DE 19928512
(43) Veröffentlichungstag der Anmeldung: 10.04.2002
(73) Patentinhaber: MERCK PATENT GmbH, 64293 Darmstadt (DE)
(72) Erfinder: BLEY, Klemens, D-64297 Darmstadt (DE)
(74) Vertreter: Weber, Dieter, Dr.
(86) Internationale Anmeldenummer: EP0005554
(87) Internationale Veröffentlichungsnummer: WO00079358

(56) Entgegenhaltungen:
- EP-A- 0 863 467
- DE-A- 4 115 355
- STN INTERNATIONAL C/O CHEMICAL ABSTRACT SERVICE: "Seminar Materials" SEARCHING REGISTRY : CHEMICAL NAMES AND MOLECULAR FORMULAS, 1. Juli 1997 (1997-07-01), Seiten 45-50, XP002157302 Columbus, OHIO, USA
- SYNOPSIS WHITE PAPER : ACCORD COMBICHEM TOOLS, [Online] 1998, XP002157303 Gefunden im Internet: <URL:www.synopsys.co.uk> [gefunden am 2001-01-15]

## Beschreibung

Die vorliegende Erfindung betrifft ein Verfahren zum Darstellen chemischer Strukturformeln auf einer Anzeigeeinrichtung mit Hilfe eines chemischen Zeichen- bzw. Grafikprogramms zum Erstellen chemischer Strukturformeln.

Entsprechende Zeichenprogramme sind bereits seit längerem bekannt. Derartige Zeichenprogramme weisen im allgemeinen einen Satz grundlegender Strukturelemente chemischer Strukturformeln auf, wobei sich jedes dieser Strukturelemente an eine gewünschte Position auf einem Bildschirm verschieben bzw. dort erzeugen läßt. Die Fläche einer entsprechenden Anzeigeeinrichtung, die zum Beispiel ein Bildschirm oder eine bedruckte Fläche sein kann, wird dabei im allgemeinen in gleiche Flächenelemente aufgeteilt und in jedem Flächenelement ist mindestens ein Strukturelement, zum Beispiel eine Linie einer Einfachbindung oder eine Doppellinie für eine Doppelbindung in einer bestimmten räumlichen Orientierung zugeordnet. Innerhalb des Flächenelementes sind die Strukturelemente im allgemeinen auch drehbar und/oder verschiebbar, damit Strukturelemente benachbarter Flächenelemente immer passend aneinandergesetzt werden können. Auch können die einzelnen Flächenelemente gegebenenfalls in Ihrer Gesamtheit drehbar sein.

Die Grundstrukturelemente für entsprechende Zeichenprogramme, die fast ausschließlich im Bereich der organischen Chemie Verwendung finden, sind daher Einfach-, Doppel- oder Dreifachbindungen in Form einer einfachen, einer doppelten oder einer dreifachen Linie, deren Enden jeweils den Ort der so verbundenen Atome oder lonen definieren. Dabei können auch mehrere entsprechende Linien unter vorgegebenen Winkeln oder auch mit kurzen Unterbrechungsstellen aneinanderhängen, wobei sich entsprechende Zeichenprogramme jedoch im allgemeinen darauf beschränken, einige wenige Grundstrukturen als komplexe Strukturelemente zur Verfügung zu stellen, z. B., einen sogenannten Benzolring, d.h. die Struktur eines regelmäßigen Sechsecks mit abwechselnden Doppel- und Einfachbindungen. Durch Anfügen weiterer Bindungslinien können daraus zwar beliebige chemische Strukturen zusammengesetzt werden, jedoch stehen solche komplexeren Strukturen im allgemeinen nicht als grundlegende Strukturelemente von vornherein zur Verfügung.

Einer üblichen Konvention entsprechend werden dabei die an den Enden der Bindungen bzw. Bindungslinien gegebenenfalls vorhandenen Kohlenstoffatome nicht ausdrücklich bezeichnet, d.h. wo zwei Linien ohne nähere Bezeichnung mit einer kurzen Unterbrechung oder unter einem Winkel < 180° aneinanderstoßen, befindet sich jeweils ein Kohlenstoffatom. Soweit jedoch andere Elemente wie Stickstoff, Wasserstoff, Sauerstoff oder Schwefel oder auch Moleküle den Abschluß einer Bindungslinie bilden, so werden die Enden solcher Linien mit entsprechenden Buchstabenkennungen entsprechend der üblichen chemischen Nomenklatur bezeichnet.

Bei der Synthese neuer chemischer Verbindungen in der chemischen Forschung und Entwicklung, teilweise aber auch bei der Analyse gegebener Substanzen sind entsprechende chemische Strukturformeln für den Chemiker ein wesentliches Hilfsmittel, da er anhand der Strukturformel wesentlich besser als anhand einer entsprechenden Summenformel oder einer, wenn auch präzisen, chemischen Bezeichnung erkennen kann, welche Abschnitte einer chemischen Struktur überhaupt für eine Reaktion mit anderen Substanzen bzw. Komponenten in Frage kommen. Auf diese Weise können gewünschte Verbindungen bzw. Strukturen wesentlich einfacher und gezielter synthetisiert werden und bestehende Strukturen anhand ihrer Bruchstücke oder auch anhand ihres Reaktionsverhaltens wesentlich leichter identifiziert werden.

Dabei geht man in der chemischen Forschung im allgemeinen in der Weise vor, daß man Strukturformeln für Ausgangssubstanzen (Edukte) und ein gegebenenfalls daraus entstehendes Produkt aufzeichnet. Da entsprechende Forschungsergebnisse auch archiviert und dokumentiert werden müssen, ist es bei den heutigen Anforderungen an die Dokumentation und die Zugänglichkeit und Recherchierbarkeit entsprechender Ergebnisse notwendig, die entsprechenden Reaktionsgleichungen möglichst mit den Strukturformeln auf elektronischem Wege zu speichem. Es versteht sich, daß hierzu die bereits existierenden Zeichen- bzw. Grafikprogramme zum Erstellen chemischer Strukturformeln im Prinzip das geeignete Instrument sind.

Allerdings ist das Erstellen entsprechender Reaktionsgleichungen bzw. der einzelnen Edukte und Produkte am Bildschirm ein oftmals aufwendiger, langwieriger und umständlicher Vorgang, wenn die Strukturen der chemischen Edukte und Produkte entsprechend umfangreich und kompliziert sind. In diesem Fall müssen nämlich sehr viele einzelne Grundelemente, die das chemische Zeichenprogramm anbietet, Stück für Stück aneinandergesetzt werden. Dies erfordert relativ viel Zeit und beinhaltet außerdem eine erhebliche Fehlerquelle, da durchaus versehentlich einzelne Strukturelement fortgelassen, hinzugefügt oder an eine falsche Position oder in einer falschen Orientierung eingefügt werden können.

Gegenüber diesem Stand der Technik liegt der vorliegenden Erfindung die Aufgabe zugrunde, ein Verfahren und ein entsprechendes System zum Darstellen chemischer Strukturformeln auf einer Anzeigeeinrichtung zu schaffen, mit deren Hilfe die Erstellung von chemischen Reaktionsgleichungen anhand von Strukturformeln wesentlich vereinfacht wird.

Hinsichtlich des Verfahrens wird diese Aufgabe dadurch gelöst, daß zu bekannten bzw. neu synthetisierten chemischen Substanzen Codierungen vergeben werden, daß die Codierungen in einem Speicher gespeichert werden, welcher mit dem Zeichenprogramm in Verbindung treten kann, wobei zu jedem Code einer chemischen Substanz auch die zugehörige Struktur oder eine Programmroutine zur Erzeugung der Struktur gespeichert werden, und daß der Code in einer lesbaren bzw. maschinenlesbaren Form auf einem Gegenstand unter eindeutiger Zuordnung zu der Substanz aufgebracht ist, so daß er von dort in einen Computer eingebbar ist, auf welchem das Zeichenprogramm läuft, wobei durch die Eingabe des Codes die Darstellung der entsprechenden Struktur auf der Anzeigeeinrichtung ausgelöst werden kann.

Bei diesem Verfahren muß also der Chemiker nicht selbst anhand einer chemischen Bezeichnung und einer Summenformel die Strukturformel einer chemischen Substanz in mühsamer Kleinarbeit zusammensetzen, sondern er gibt lediglich einen der Substanz zugeordneten Code in einen entsprechenden Computer ein, wobei dann durch das Zeichenprogramm sofort die vollständige Struktur dieser Substanz entweder aus einem Speicher abgerufen oder durch eine dem Code zugeordnete Programmroutine erzeugt wird.

Es versteht sich, daß der Code in irgendeiner lesbaren Form vorliegen muß, damit er in den Computer eingegeben werden kann, wobei unter "lesbar" sowohl von Personen lesbare als auch maschinenlesbare Codierungen verstanden werden, wie zum Beispiel die bekannten Barcodes (Strichcodes) oder aber auch eine codierte Speicherung in magnetischer Form auf einem Magnetstreifen oder auch auf entsprechenden optischen Speichern. In Frage kämen durchaus auch maschinelle Klarschriftleser, die auf eine Fläche aufgedruckte Ziffern korrekt lesen können. Durch Verwendung eines entsprechenden Lesegerätes kann auch die Eingabe des Codes nochmals beträchtlich vereinfacht werden, so daß innerhalb von Sekundenbruchteilen die gewünschte Struktur zum Beispiel auf einem Bildschirm erscheint, ausgedruckt werden kann oder in einer sonstigen Weise für eine Darstellung verfügbar gemacht wird.

Dabei ist es selbstverständlich zweckmäßig, wenn der Code auf einem Behälter einer chemischen Substanz, auf einem Etikett hierzu, einem Beipackzettel oder einem Tabellenwerk aufgebracht ist. Zweckmäßig wäre es also, wenn in einem chemischen Labor jeder Behälter, in welchem eine chemische Substanz aufbewahrt wird, die als Edukt einer chemischen Reaktion in Frage kommt, mit einer entsprechenden, aufgedruckten Codierung versehen ist. Dabei können zum Beispiel ohnehin bereits vorhandene Artikelnummem, die eine chemische Substanz eindeutig kennzeichnen, als Codierungen verwendet werden. Wie bereits erwähnt, können auch Barcodes auf einen Behälter bzw. einem auf dem Behälter aufgebrachten Etikett aufgedruckt sein. Notfalls kann aber auch ein Tabellenwerk vorgesehen werden, in dem neben einer üblichen chemischen Bezeichnung einer Substanz auch eine entsprechende Codierung angegeben ist, zum Beispiel in Form eines Barcodes, so daß man lediglich mit dem Lesegriffel oder dergleichen eines Barcodelesers über diesen gespeicherten Barcode neben einer in üblicher chemischer Nomenklatur angegebenen Substanz hinwegfahren muß, um die gewünschte Eingabe in einen Computer vorzunehmen, woraufhin die betreffende Struktur zum Beispiel auf einem Bildschirm erscheint.

Im Falle von Gemengen, Lösungen, Emulsionen oder Mischungen von Substanzen, die chemisch nicht miteinander reagieren, wohl aber jeweils mit einem anderen (gemeinsamen) Edukt reagieren können, wird zweckmäßigerweise die Struktur der einzelnen Komponenten mit einem "+"-Zeichen dazwischen allein auf der Basis der entsprechenden Codierung für das Gemenge, die Lösung, Emulsion oder Mischung von Substanzen auf der Anzeigeeinrichtung wiedergegeben.

Werden ansonsten mehrere getrennt (in Behältern) aufbewahrte Edukte zusammengebracht, die unter Bildung eines oder mehrerer neuer Produkte miteinander reagieren, so kann man nacheinander die verschiedenen Codierungen der Edukte eingeben, so daß sie nacheinander in einer Reihe auf einem Bildschirm oder dergleichen erscheinen und gegebenenfalls durch Pluszeichen verbunden sind. Soweit aus den Ausgangsprodukten bzw. -edukten die Endprodukte bereits ablesbar sind und sich zum Beispiel nur in Einzelheiten von einem der Edukte unterscheiden, ist es zweckmäßig, dieses Edukt nochmals auf der rechten Seite einer chemischen Reaktionsgleichung wiederzugeben und lediglich noch die erforderlichen Modifikationen vorzunehmen. Dabei ist vorausgesetzt, daß das so entstandene Produkt neu ist und daß noch keine Codierung für diese neue Struktur besteht, so daß dann eine entsprechende neue Codenummer für die neue Substanz vergeben werden muß. Sollte jedoch das Produkt an sich bereits bekannt sein, so wäre es gegebenenfalls auch möglich, aus einem Tabellenwerk die zugehörige Codierung aufzurufen, um dadurch die Darstellung nochmals zu vereinfachen.

Eine entsprechendes System mit einer Vorrichtung zur Durchführung eines solchen Verfahrens, welche aus einem Computer und einem darauf installierbaren bzw. installierten Zeichen- bzw. Grafikprogramm zur Darstellung chemischer Strukturformeln besteht, und welche auch einen entsprechenden Speicher hat, ist zur Lösung der oben formulierten Aufgabe dadurch gekennzeichnet, daß der Speicher für die Speicherung von Codes ausgelegt ist und daß weiterhin Speicherplatz für den Codes zugeordnete chemische Strukturen bzw. für die Darstellung dieser Strukturen erzeugende Programmroutinen vorgesehen sind, wobei Eingabeeinrichtungen zur Eingabe der Codes vorgesehen sind und die Codes in einer lesbaren Form und in eindeutiger Zuordnung zu den zugehörigen chemischen Substanzen bereitgehalten werden.

Die Eingabeeinrichtungen bestehen im einfachsten Fall aus einer Tastatur, über welche nur die Codierung eingegeben zu werden braucht, oder noch bevorzugter einer Maus, mit deren Hilfe man zum Beispiel durch Anwählen (Anklicken) in einem elektronisch gespeicherten Tabellenwerk die gewünschte chemische Strukturformel erzeugen kann. Bei der Eingabeeinrichtung kann sich jedoch auch um einen Klarschriftleser, einen Barcodeleser oder einen sonstigen optischen oder einen magnetischen Lesekopf handeln, je nachdem in welcher Form die Codes gespeichert sind. Dabei ist lediglich darauf zu achten, daß die Codes eindeutig bestimmten chemischen Substanzen zugeordnet sind, was, wie bereits erwähnt, am einfachsten dadurch geschieht, daß die Codes auf Behälter aufgedruckt sind, die die zugehörige chemische Substanz enthalten.

Weitere Vorteile, Merkmale und Anwendungsmöglichkeiten der vorliegenden Erfindung werden deutlich anhand der folgenden Beschreibung einer bevorzugten Ausführungsform und der dazugehörigen Figuren.

Es soll beispielsweise eine Reaktionsgleichung in Form von Strukturformeln dargestellt werden, bei welcher aus der Umsetzung von Anilin mit Acetylchlorid Acetanilid entsteht.

Dabei sei für die Zwecke der vorliegenden Beschreibung einmal angenommen, daß zu den beiden Edukten Anilin und Acetylchlorid entsprechende Codes bereits vergeben und gespeichert sind, wohingegen Acetanilid die Rolle einer neuen, noch unbekannten Substanz einnehmen soll, deren Bildung man jedoch anhand der chemischen Strukturformeln der Edukte anschaulich leicht nachvollziehen kann.

In diesem Fall wird zum Beispiel über einen Barcodeleser von einer Anilinflasche, auf deren Etikett bzw. Außenseite ein entsprechender Barcode aufgedruckt oder -geprägt ist, der Code des Anilins eingegeben. Damit erscheint die folgende Struktur, wobei der Pfeil, der die rechte Seite von der linken Seite einer Reaktionsgleichung trennt, automatisch erzeugt wird:

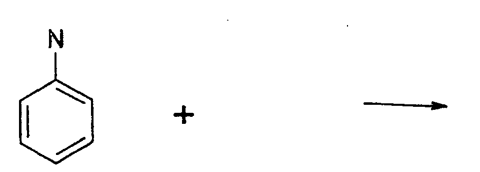

Da sich Acetanilid in der chemischen Struktur nur an wenigen Stellen von Anilin unterscheidet, ist es zweckmäßig, für die Darstellung auf der rechten Seite der Reaktionsgleichung nochmals den Code für Anilin aufzurufen, so daß man, von dieser Struktur ausgehend, lediglich noch die Modifikationen vornehmen muß, die hieraus das bereits erwähnte Acetanilid machen. Diese Zwischenstufe ist in der nachstehenden Gleichung mit Hilfe der entsprechenden Strukturformeln dargestellt

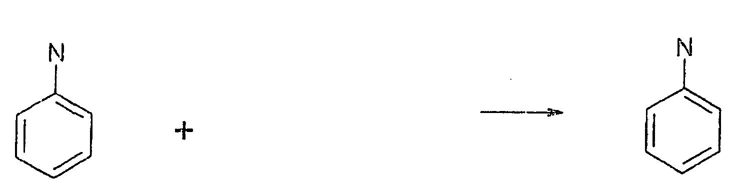

Anschließend wird auf der linken Seite noch neben einem "+"-Zeichen der Code von Acetylclorid aufgerufen und die Struktur dort dargestellt, so daß sich zunächst das folgende Bild ergibt.

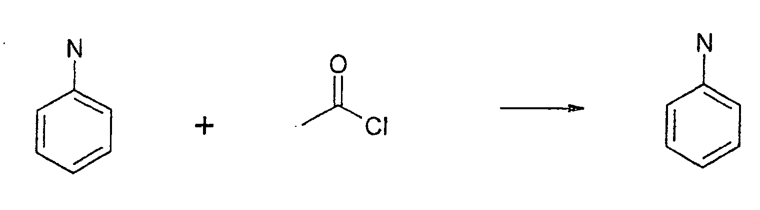

Nunmehr müssen noch auf der rechten Seite die notwendigen Ergänzungen vorgenommen werden. Auch hierzu kann zunächst nochmals die Struktur von Acetytclorid als Anhängsel an die bereits bestehende Anilinstruktur angesetzt werden und die abschließende Korrektur erfolgt dann lediglich durch Entfernen der Buchstaben Cl für Chlor aus dieser Struktur und der Eingabe des zusätzlichen Produktes CIH, wobei die Angabe nicht interessierender Nebenprodukte auch unterbleiben kann.

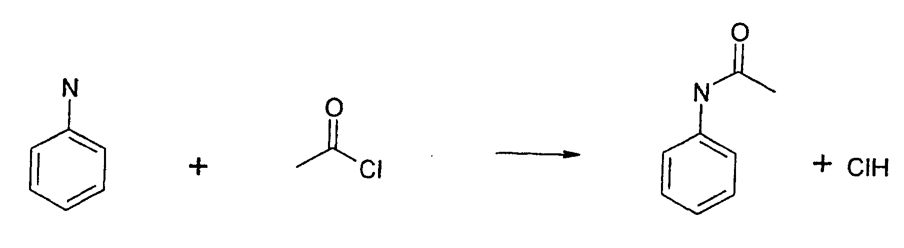

Es versteht sich, daß das vorstehende Beispiel ein ausgesprochen einfaches Beispiel ist, bei welchem der Zeitgewinn gegenüber dem Aufbau der einzelnen Strukturen innerhalb eines normalen chemischen Zeichenprogramms noch nicht sehr deutlich zutage tritt. Die Edukte können aber auch wesentlich komplizierter sein und aus einer Vielzahl von Benzolringen, Ketten und anderen Strukturen zusammengesetzt sein, so daß beim Aufrufen einer Codenummer für eine in ihrer chemischen Struktur entsprechend kompliziertere Substanz eine beträchtliche Vereinfachung darstellt. Die Reihenfolge der in dem obigen Beispiel dargestellten Schritte kann selbstverständlich geändert werden, zum Beispiel in der Weise, daß die Reaktionsgleichung Schritt für Schritt von links nach rechts aufgebaut wird.

Dabei ist es im übrigen zweckmäßig, wenn das System so aufgebaut ist, daß grundsätzlich alle bereits bekannten chemischen Substanzen als auch neue synthetisierte chemische Substanzen mit ihren entsprechenden Strukturformeln und einem zugehörigen Code so schnell wie möglich in einer zentralen Datenbank gespeichert werden, wobei ein lokaler Computer dann lediglich eine entsprechende Verbindung zu der Datenbank haben muß, die Daten selbst jedoch anderweitig verwaltet werden können.

Auch ohne weitere Ausführungen wird davon ausgegangen, daß ein Fachmann die obige Beschreibung im weitesten Umfang nutzen kann. Die bevorzugten Ausführungsformen und Beispiele sind deswegen lediglich als beschreibende, keineswegs als in irgendeiner Weise limitierende Offenbarung aufzufassen.

## Patentansprüche

1. Verfahren zum Darstellen chemischer Strukturformeln auf einer Anzeigeeinrichtung mit Hilfe eines chemischen Zeichen- bzw. Grafikprogramms zum Erstellen chemischer Strukturformeln, **dadurch gekennzeichnet, daß** zu bekannten bzw. neu synthetisierten chemischen Substanzen Codierungen vergeben werden, daß die Codierungen in einem Speicher gespeichert werden, welcher mit dem Zeichenprogramm verbindbar ist, wobei zu jedem Code einer chemischen Substanz auch die zugehörige Struktur oder eine Programmroutine zur Erzeugung der Struktur gespeichert werden, und daß der Code in einer lesbaren oder maschinenlesbaren Form auf einem Gegenstand unter eindeutiger Zuordnung zu der Substanz aufgebracht wird, so daß er von dort in einen Computer eingebbar ist, auf welchem das Zeichenprogramm läuft, wobei durch die Eingabe des Codes die Darstellung der entsprechenden Struktur auf der Anzeigeeinrichtung auslösbar ist.

2. Verfahren nach Anspruch 1, **dadurch gekennzeichnet, daß** der Code auf einem Behälter, Etikett, Beipackzettel oder einem Tabellenwerk aufgebracht ist.

3. Verfahren nach Anspruch 1 oder 2, **dadurch gekennzeichnet, daß** der Code als Ziffemfolge, als Barcode oder auf einem magnetischen oder optischen Speichermedium aufgebracht ist.

4. System zur Darstellung chemischer Strukturformeln mit einer Vorrichtung zur Durchführung des Verfahrens nach einem der Ansprüche 1 bis 3, mit einem Computer und einem darauf installierbaren Zeichen- bzw. Grafikprogramm zur Darstellung chemischer Strukturformeln und mit mindestens einem Speicher, **dadurch gekennzeichnet, daß** der Speicher für die Speicherung von Codes ausgelegt ist und daß weiterhin Speicherplatz für den Codes zugeordnete chemischeStruktuten bzw. für die Darstellung dieser Strukturen erzeugende Programmroutinen vorgesehen sind, wobei Einrichtungen zur Eingabe der Codes vorgesehen sind und wobei die Codes und die zugeordneten Strukturen in eindeutiger Zuordnung zu den entsprechenden chemischen Substanzen in lesbarer Form bereitgehalten werden.

5. System nach Anspruch 4, **dadurch gekennzeichnet, daß** als Einrichtung zur Eingabe der Codes ein Barcodeleser, ein Klarschriftleser oder ein Magnetstreifenleser vorgesehen ist.

6. System nach Anspruch 4 oder 5, **dadurch gekennzeichnet, daß** die Codes auf Behältern, Etiketten oder Beipackzetteln für die jeweiligen chemischen Substanzen aufgebracht sind oder in einem Tabellenwerk gespeichert und nachschlagbar sind.

## Claims

1. A method of representing chemical structural formulae on a display device by means of a chemical symbol or graphic program for producing chemical structural formulae, **characterised in that** codings are allocated to known or newly synthesised chemical substances, that the codings are stored in a memory which can be connected to the symbol program, wherein in relation to each code of a chemical substance the associated structure or a program routine for production of the structure are also stored, and that the code is applied in a readable or machine-readable form to an article with unequivocal association with the substance so that from there it can be inputted into a computer on which the symbol program is running, wherein by virtue of the input of the code the representation of the corresponding structure on the display device can be triggered.

2. A method according to claim 1 **characterised in that** the code is applied to a container, label, instruction leaflet or list of contents or a table work.

3. A method according to claim 1 or claim 2 **characterised in that** the code is applied in the form of a sequence of digits, a bar code or to a magnetic or optical storage medium.

4. A system for representing chemical structural formulae having an apparatus for carrying out the method according to one of claims 1 to 3 comprising a computer and a symbol or graphic program installable thereon for the representation of chemical structural formulae and at least one memory, **characterised in that** the memory is designed for the storage of codes and that in addition there is provided storage space for chemical structures associated with the codes or for program routines producing the representation of said structures, wherein there are provided input devices for input of the codes and the codes and the associated substances are held in readiness in unequivocal association with the corresponding chemical substances in readable form.

5. A system according to claim 4 **characterised in that** a bar code reader, a plain text reader or a magnetic strip reader is provided as the device for inputting the codes.

6. A system according to claim 4 or claim 5 **characterised in that** the codes are applied to containers, labels or instruction leaflets or lists of contents for the respective chemical substances or are stored and can be looked up in a table work.

## Revendications

1. Procédé de représentation de formules chimiques développées sur un dispositif de présentation à l'aide d'un programme de représentation graphique resp. de traçage de substances chimiques en vue de présenter des formules chimiques développées, **caractérisé en ce que** des codes sont attribués à des substances chimiques connues ou nouvellement synthétisées, les codes sont stockés dans une mémoire qui peut être liée à un programme de traçage, pour chaque code d'une substance chimique, la structure associée ou bien un sous-programme est mémorisé en vue de générer la structure, et le code est appliqué, sous forme lisible directement ou par une machine, sur un objet en étant associé de façon univoque à la substance de façon à pouvoir l'entrer ensuite dans un ordinateur sur lequel tourne le programme de traçage, l'entrée du code permettant le déclenchement de la représentation de la structure correspondante sur le dispositif de présentation.

2. Procédé selon la revendication 1, **caractérisé en ce que** le code est appliqué sur un récipient, une étiquette, une notice explicative ou introduit dans une table.

3. Procédé selon la revendication 1 ou 2, **caractérisé en ce que** le code est appliqué sous la forme d'une suite de chiffres, d'un code à barre, ou sur un support de stockage magnétique ou optique.

4. Système de représentation de formules chimiques développées au moyen d'un dispositif de mise en oeuvre du procédé selon l'une des revendications 1 à 3, lequel dispositif comporte un ordinateur sur lequel est installé un programme de représentation graphique resp. de traçage et au moins une mémoire, **caractérisé en en ce que** la mémoire est destinée au stockage de codes et en ce qu'il est prévu en outre de la place en mémoire pour les structures chimiques associées aux codes resp. pour les sous-programmes générant la représentation de ces structures, des dispositifs étant prévus pour entrer les codes, et les codes et les structures associées sont disponibles sous forme lisible associée de façon univoque aux substances chimiques correspondantes.

5. Système selon la revendication 4, **caractérisé en ce qu'**il est prévu comme dispositif d'entrée des codes un lecteur d'écriture en clair ou bien un lecteur de bande magnétique.

6. Système selon la revendication 4 ou 5, **caractérisé en ce que** les codes sont appliqués sur des récipients, des étiquettes ou des notices explicatives destinés aux substances chimiques correspondantes ou bien sont mémorisés dans une table pouvant être consultée.
